# EUROPEAN PATENT APPLICATION

(11) **EP 2 113 203 A1**
(43) Date of publication of application: **04.11.2009**
(21) Application number: 09251190.6
(22) Date of filing: 27.04.2009
(51) Int. Cl.: A61B 10/00, B01L 3/00

(54) **Fluid collection device with expresser plug holder**

(30) Priority: 29.04.2008 US 111788
(71) Applicant: Varian, Inc., Palo Alto, CA 94304-1030 (US)
(72) Inventor: Grenz, Robert Lee, Santa Anna California 92702 (US); Galloway, Robert Keith, Raleigh North Carolina 27617 (US); Roberts, Roger Quinn, Dana Point California 92629 (US); Gamboa, Lorna Arellano, San Clemente California 92673 (US); Huynh, Nina Thao, Ct. Orange California 92869 (US); Schultheis, Stephen K., Ct. Laguna Hills California 92653 (US)
(74) Representative: Foster, Mark Charles

(57) **Abstract**

A fluid collection device has a collection vial (14) with a reservoir chamber (36) for receiving a fluid sample. An elongated expresser (28) is disposed within the collection via (1), which includes a fluid permeable base (30) enabling passage of fluid sample into the reservoir chamber. A fluid test card (48) is supported by an expresser bottom flange (40) within the collection vial and a plunger (58) is provided for compression of a swab (66) saturated with fluid specimen into the reservoir chamber. A cap (70) is provided for engaging the collection vial in order to seal the compressor, fluid test card, compressed swab, and plunger within the collection vial.

## Description

### FIELD OF THE INVENTION

The present invention generally relates to fluid specimen collection and testing devices and is more specifically directed to oral fluid or saliva collection and testing devices.

### BACKGROUND OF THE INVENTION

Sampling and testing of body fluids is common for both testing and monitoring the human body for any number of biochemical or physiological conditions and, of course, for determining the general state of health of an individual.

Use of saliva as a testing medium is often preferable due to its availability without intrusive methods such as is required in sampling of blood. Urine samples, although non invasive, require patient monitoring during sampling to insure integrity. Accordingly, the use of saliva as a medium in biochemical and physiological analysis is desirable since it can be obtained by non-invasive methods and is easily monitored.

In addition, saliva has been found to be a very reliable carrier of analyte indicators which are useful for the qualitative detection of amphetamines, methamphetamine, cocaine, opiates, THC and their metabolites and other drugs of misuse and substances along with applications including nucleic acid markers and vial infections such as HIV.

Although saliva may be advantageously used in the analysis of a patient's biochemistry, problems still remain in the collection of saliva and in the handling thereof by laboratory technicians. As in the testing of any body fluid, minimum exposure due to handling is of utmost importance.

Prior art devices include Varian, Inc.'s Ora Lab products as set forth in U.S. Patent No. 7,282,181.

The present invention improves upon prior art devices by providing a device that includes a collection vial with a reservoir chamber enclosed therein for collecting samples.

### SUMMARY OF THE INVENTION

A fluid collection device in accordance with the present invention generally includes a collection vial having an open end and a closed bottom with a reservoir chamber for receiving a fluid sample.

An elongated expresser is insertable into the collection vial and includes a fluid permeable base enabling passage of a fluid sample into the reservoir chamber and a bottom flange. A fluid test card is provided, which is supported by the bottom flange and includes an end for absorbing the sample fluid from the reservoir chamber.

A plunger with a piston and grip is sized for reception by the expresser and a swab fixable to the plunger piston is provided for absorbing the fluid sample to be tested and expressing the fluid sample through the expresser base upon compression by the piston.

A cap is provided for engaging the collector vial open end in a conventional manner in order to seal the expresser, fluid test card, swab and plunger within the collection vial.

Upon assembly, the fluid collected by the swab is expressed out of the swab and into the reservoir chamber as the swab is pressed into the expresser.

Preferably, the plunger has a length causing the swab to remain compressed during cap engagement with the collection vial. The swab is compressed against the expresser base and further seals the fluid sample within the reservoir chamber.

In this manner, the fluid, such as saliva, expressed from the swab into the reservoir chamber remains contained within the reservoir chamber during handling and/or transit.

Also, the plunger does not have to be removed from the expresser as is the case with prior art devices. This eliminates any smearing or dripping of sample.

An additional advantage of maintaining the fluid sample within the reservoir chamber is that the surface area of the reservoir chamber is extremely reduced compared with the surface area of the much larger collection vial. By maintaining the sample within the smaller enclosed volume of the reservoir chamber, the reaction of the fluid sample with the container is substantially reduced.

Because components within the fluid sample are prevented from reacting or binding with the container, the components of the fluid remain available for testing, and therefore performance and reliability of the testing device is enhanced.

Preliminary test results are observable through the use of the test card while the fluid sample may also be stored or transported to a laboratory without the need for buffering agents to maintain the sample in condition to provide reliable test results. By eliminating the need for buffering agents, the variability and inconsistency on testing that is created by the buffering agents can be removed to thereby improve reliability of the test device.

More particularly, upon assembly of the expresser within the collection vial, the bottom flange is suspended above the vial bottom to define the reservoir chamber. In addition, the bottom flange may have a slot therethrough and the test card end may include a tongue which is sized for insertion through the bottom flange slot. In this manner, the majority of the test card can be held in a position above the reservoir bottom for observation through the collection vial.

The vial may include a tapered inside diameter, narrower toward the vial bottom, and the bottom flange has dimensions for engaging the vial inside diameter proximate to the vial bottom. In this manner, the bottle flange is suspended above the collection vial bottom as hereinabove noted.

The expresser may be further provided with a top flange that aids in positioning the expresser within the collection vial. Advantageously, the top flange may be shaped to provide an opening or gap relative to the collection vial that allows for the insertion or placement of a test card into the collection vial. A test card may therefore be placed into the collection vial adjacent to the expresser with the tongue portion of the test strip inserted through the slot in the bottom flange of the expresser as described above.

More particularly, in accordance with the present invention, the permeable base of the expresser may include a plurality of spaced apart ribs and the cap may include a bearing surface for engaging the plunger grip or forcing the plunger and the piston within the vial upon cap engagement with the vial top in order to compress the swab against the expresser base.

### BRIEF DESCRIPTION OF THE DRAWINGS

The advantages and features of the present invention will be better understood by the following description when considered in conjunction with the accompanying drawings, in which:

Figure 1 is an exploded perspective view of the present invention generally showing a vial, expresser, plunger, and cap;

Figure 2 is a cross section view of the invention upon assembly showing the compression of a swab disposed at the base of the expresser;

Figure 3 is a cross-sectional view taken along the line 3-3 of Figure 2;

Figure 4 is a perspective view of the plunger showing a piston and a grip;

Figure 5 is a perspective view of the expresser showing top and bottom flanges; and

Figure 6 is a plan view of a test card with a tongued end for being supported by the expresser bottom flange.

### DETAILED DESCRIPTION

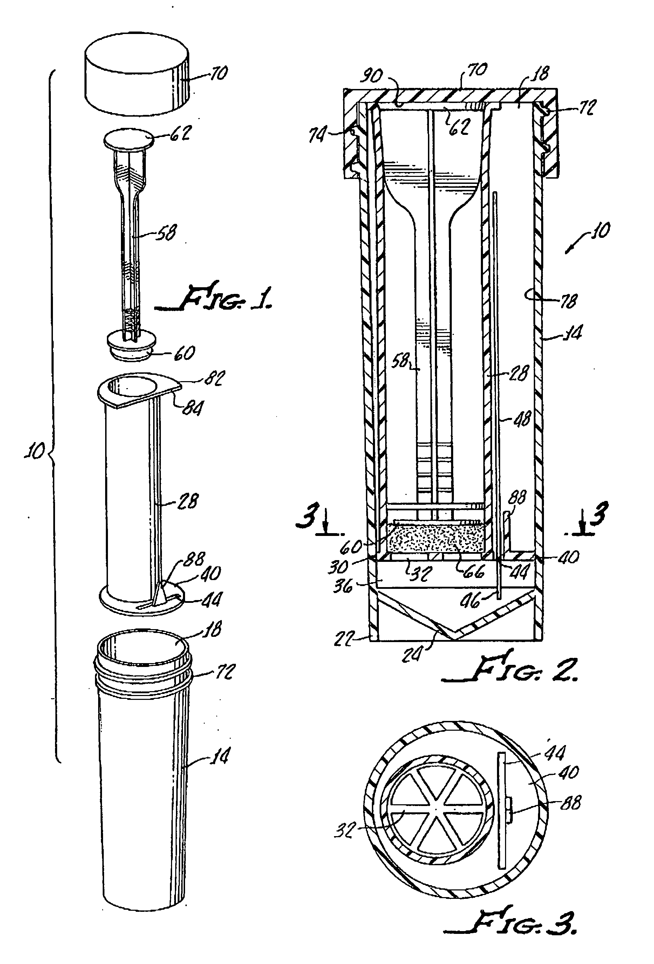

With reference to Figures 1-6, there is shown a fluid collection device 10 in accordance with the present invention generally illustrating a collection vial 14 having an open end 18 and a closed bottom 22 with a conical portion 24. An elongated expresser 28, sized for insertion into the collection vial 14 includes a fluid permeable base 30, which may include spaced apart ribs 32 (shown also in figure 3) enabling passage of fluid sample into a reservoir chamber 36 defined by a bottom flange 40 and the vial conical portion 24. The bottom flange 40 may include a slot 44 for engaging the tongue 46 of a test card 48.

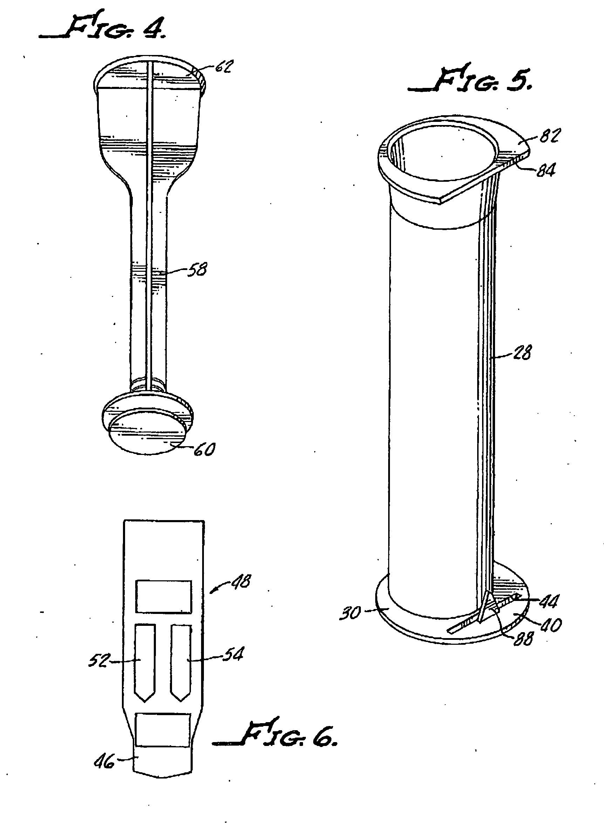

The end, or tongue, 46 of the test card 48 thus extends into the reservoir chamber 36 for the absorption of fluid sample. The card 48 may be a conventional lateral flow chromatographic immunoassay device having test strips 52, 54 (as seen in Figure 6) for wicking sample fluid from the reservoir chamber 36. Results of the test of wicked fluid may be observed directly through the transparent vial 14.

A plunger 58 having a piston 60 and a grip 62 is sized for reception by the elongated expresser 28 as illustrated in Figures 1-2. One of skill in the art will recognize that the collection vial 14, as well as the expresser 28 may comprise any number of suitable cross-sections. One embodiment as discussed in more detail here is a circular cross-section for the collection vial 14 and the elongated tubular portion of the expresser.

An absorbent swab 66 of any suitable material is either fixed, or fixable, to the plunger piston 60 for collection of a fluid sample, for example, from an oral cavity with such collection being facilitated by the plunger 58.

After insertion of the plunger 58 into the expresser 28, the fluid sample is expressed, or squeezed from the swab 66 by compression against the ribs 32 of the bottom flange 40. A cap 70, including threads 74 for engaging vial threads 72 (or any other acceptable pressed-fit clasp or other cap closure), is utilized for sealing the open end 18 of the vial 14 in order to enclose and seal the expresser 28, fluid test card 48, swab 66 and plunger 58 within the collection vial 14.

Importantly, the plunger 58 has a length causing the swab 66 to remain compressed against the bottom flange 40 during cap 70 engagement with the vial 14 open end 18 in order to seal the reservoir chamber 36 with concomitant advantages as hereinbefore described.

As shown in Figure 2, upon assembly the expresser 28 bottom flange 40 is suspended above the conical portion 24 of the collection vial 14, which defines the reservoir chamber 36. This may be accomplished by providing the collection vial 14 with a tapered inside surface 78, which in combination with bottom flange 40 dimensions, causes engagement of the vial inside diameter, or surface, 78 with the flange 40 proximate the vial bottom. Alternative embodiments may include, for example, using a constant cross section collection vial 14 that includes a rib around its interior perimeter at the desired level, upon which the bottom flange 40 may rest. According to a second alternative, the bottom flange 40 of the expresser 28 may rest upon the outer perimeter of the conical portion 24 at the bottom of the collection vial 14.

As shown in Figure 1, the expresser 28 further includes a radial extending top flange 82 with a flat portion 84 for supporting the test card 48 within the vial 14 and for providing access for insertion of the test card 48 into the collection vial 14, as illustrated in Figure 2. In that regard, a finger 88, upstanding from the bottom flange 40, may be provided adjacent the slot 44 for further support and for facilitating insertion of the test card 48, tongue 46 into the slot 44 during assembly.

With reference again to Figure 2, the cap 70 includes an inside bearing surface 90 for engagement with the plunger grip 62 in order to compress the swab 66 via the piston 60 upon assembly of the fluid collection device 10.

It should be appreciated that while the collection vial 14, reservoir chamber 36, and expresser 28 have been described as individual components, they may be integrated and formed with a single piece construction of a suitable material. Alternatively, the collection vial 14 may be a single piece of construction and the reservoir chamber and expresser may be an integrated single piece construction which is sized to fit within the collection vial 14.

The use of the present invention is performed by instructing a test subject to saturate the swab 66 with saliva and thereafter inserting the swab 66 through the open end 18 of the collection vial 14 and into the expresser 28. Thereafter, the swab 66 is compressed by the piston 60 against the bottom flange 40 by either pushing the grip 62 with one's fingers or pressing the swab 66 utilizing the cap 70. The cap 70 is then secured to the top 48 of the collection vial 14 and after waiting for the test to complete, reading the test results from the test card 48 through the vial 14. The method may further include shipping or transport of the sealed vial to a separate testing area.

Although there has been hereinabove described a specific fluid collection device with expresser plug holder in accordance with the present invention for the purpose of illustrating the manner in which the invention may be used to advantage, it should be appreciated that the invention is not limited thereto. That is, the present invention may suitably comprise, consist of, or consist essentially of the recited elements. Further, the invention illustratively disclosed herein suitably may be practiced in the absence of any element which is not specifically disclosed herein. Accordingly, any and all modifications, variations or equivalent arrangements which may occur to those skilled in the art, should be considered to be within the scope of the present invention as defined in the appended claims.

## Claims

1. A fluid collection device comprising:
a collection vial having an open end and a closed bottom with a reservoir chamber for receiving a fluid sample;
an elongated expresser disposed in said collection vial, having a fluid permeable base enabling passage of the fluid sample into said reservoir chamber, and a bottom flange disposed above said closed bottom;
a plunger, having a piston and a grip, sized for reception by said expresser;
a swab, fixable to the plunger piston, for absorbing the fluid sample and expressing the fluid sample through the expresser base upon compression by said piston; and
a cap for engaging the collection vial open end in order to seal the expresser, fluid test card, swab and plunger within said collection vial.

2. The fluid collection device of claim 1, wherein said plunger has a length causing said swab to remain compressed during cap engagement with said collection vial, said swab being compressed against the expresser base and sealing the fluid sample within said reservoir chamber.

3. The fluid collection device of claim 1 or 2, further comprising a fluid test card supported by the bottom flange and having an end for absorbing a portion of the fluid sample from said reservoir chamber.

4. The fluid collection device of claim 3, wherein said bottom flange has a slot therethrough and the test card end includes a tongue sized for insertion through the bottom flange slot.

5. The fluid collection device of claim 4, wherein said elongated expresser has a substantially circular cross section.

6. The fluid collection device of claim 5, wherein said expresser further comprises a radially extending top flange having a flat portion, aligned with the bottom flange slot for supporting said test card.

7. The fluid collection device of claim 1,2,3,4,5 or 6, wherein the vial includes a tapered inside diameter, narrower toward the vial bottom, and the bottom flange has dimensions for engaging the vial inside diameter proximate the vial bottom.

8. The fluid collection device of any one of the preceding claims, wherein the closed bottom of the vial includes a conical portion and the bottom flange engages the conical portion of the vial.

9. The fluid collection device of any one of the preceding claims, wherein said fluid permeable base includes a plurality of spaced apart ribs.

10. The fluid collection device of any one of the preceding claims, wherein said cap includes a bearing surface for engaging the plunger grip for forcing said plunger and piston within the vial upon cap engagement with the vial top in order to compress the swab against the expresser base.
